# EUROPEAN PATENT APPLICATION

(11) **EP 4 582 452 A1**
(43) Date of publication of application: **09.07.2025**
(21) Application number: 23860837.6
(22) Date of filing: 29.08.2023
(51) Int. Cl.: C07K 16/28, C07K 14/725, A61P 35/00, A61K 35/17

(54) **NOVEL ANTI-PD-L1 CHIMERIC ANTIGEN RECEPTOR, AND IMMUNE CELLS EXPRESSING SAME**

(30) Priority: 02.09.2022 KR 20220111672
(71) Applicant: Korea Research Institute of Bioscience and Biotechnology, Daejeon 34141 (KR)
(72) Inventor: KIM, Tae-Don, Daejeon 34141 (KR); LEE, Sooyun, Daejeon 34141 (KR)
(74) Representative: Berggren Oy
(86) International application number: PCT/KR2023/012785
(87) International publication number: WO 2024/049161

(57) **Abstract**

The present invention relates to a novel antibody or an antigen-binding fragment thereof, which specifically binds to PD-L1, a chimeric antigen receptor comprising an antigen-binding variable fragment of the antibody, immune cells expressing the chimeric antigen receptor, and a pharmaceutical composition for treating or preventing cancer, the pharmaceutical composition comprising the immune cells.

## Description

### TECHNICAL FIELD

### [CROSS-REFERENCE TO RELATED APPLICATIONS]

This present application is based on and claims priority to Korean Patent Application No. 10-2022-0111672, filed on September 2, 2022 in the Korean Intellectual Property Office, the disclosure of which is incorporated herein by reference in its entirety.

### [TECHNICAL FIELD]

The present invention relates to a novel antibody or an antigen-binding fragment thereof that specifically binds to PD-L1, a chimeric antigen receptor comprising the antigen-binding fragment of the antibody, an immune cell expressing the chimeric antigen receptor, and a pharmaceutical composition comprising the immune cell.

### BACKGROUND ART

The methods for treating cancer have been steadily developed and changed, methods such as a surgical operation, chemotherapy, and radiotherapy have been continuously used so far, but these existing methods for treating cancer are mostly effective only in the initial state in which the cancer has not metastasized, and there is a limitation in that, in a state in which the metastasis has already progressed, for example, even when a surgical operation is performed, there is high probability of recurrence in the future. Accordingly, recently, studies on a method for using an immune response to treat cancer have been continued.

Among them, there is a growing interest in a cell therapy method in which immune cells are used to enhance or genetically modify them and the resulting immune cells are injected back into a patient, and for example, technologies for a tumor infiltrating lymphocyte (TIL), a chimeric antigen receptor (CAR), and a T-cell receptor (TCR) have been studied. In particular, the chimeric antigen receptor, which is an artificial receptor designed to deliver antigen specificity to T cells or natural killer cells (NK cells), contains an extracellular binding domain capable of activating the immune cells and providing specific immunity by binding of the receptor to a cancer cell-specific antigen, a transmembrane domain, and an intracellular signaling domain. In addition, T cells expressing such a chimeric antigen receptor were named CAR-T cells (Kershaw et al., Nat. Rev. Immunol., 5(12): 928-940 (2005); Restifo et al., Nat. Rev. Immunol., 12(4): 269-281 (2012)), and natural killer cells were named CAR-NK cells.

The intracellular signaling domain of the chimeric antigen receptor is mainly based on an intracellular signaling domain of CD3zeta, which is a signaling subunit of a T cell receptor (first-generation CAR). In addition, the intracellular signaling domain has evolved to add an intracellular signaling domain of a co-stimulatory molecule that promotes the growth and differentiation of the immune cells. For example, CAR-T cell therapeutic agents currently on the market use the intracellular signaling domains of CD28 and 4-1BB co-stimulatory molecules, respectively (second-generation CAR), and thereafter a CAR containing both CD28 and 4-1BB intracellular signaling domains (third-generation CAR) have been attempted (Stegen et al., Nat. Rev. Drug Discov., 14 (7): 499-509 (2015)).

Meanwhile, programmed cell death ligand 1 (PD-L1) is a protein expressed from human CD274 gene, is known to be overexpressed in various kinds of tumor cells, including breast cancer and lung cancer, and inhibits an immune response of an immune cell by binding to PD-1 protein expressed on the surface of the immune cell. Under such a technical background, there is a need to study other strategies and methods for treating cancer by developing antibodies targeting PD-L1 that is particularly overexpressed in cancer cells or CAR-NK and CAR-T therapeutic agents using the same.

### DISCLOSURE OF THE INVENTION

### TECHNICAL PROBLEM

An object of the present invention is to provide an antibody or an antigen-binding fragment thereof that may specifically bind to PD-L1 that is mainly expressed in cancer cells.

In addition, another object of the present invention is to provide a novel chimeric antigen receptor that may amplify cytotoxicity or cytolytic activity against cancer cells when expressed in an immune cell.

In addition, still another object of the present invention is to provide a polynucleotide and an expression vector for expressing the chimeric antigen receptor.

In addition, still another object of the present invention is to provide an immune cell having an excellent effect of treating cancer by expressing the chimeric antigen receptor on a surface thereof.

In addition, still another object of the present invention is to provide a pharmaceutical composition for treating cancer using the immune cell.

### TECHNICAL SOLUTION

In order to achieve the above objects, an aspect of the present invention provides an antibody or an antigen-binding fragment thereof that specifically binds to programmed death ligand 1 (PD-L1), the antibody or the antigen-binding fragment thereof including: a heavy chain variable region containing a heavy chain CDR1 containing an amino acid sequence of X1-Y-X2-M-X3 (SEQ ID NO: 1), a heavy chain CDR2 containing an amino acid sequence of X4-I-S-X5-S-G-X6-X7-X8-Y-Y-A-D-S-V-K-G (SEQ ID NO: 2), and a heavy chain CDR3 containing any one sequence selected from the group consisting of SEQ ID NO: 3, SEQ ID NO: 4, SEQ ID NO: 5, SEQ ID NO: 6, and SEQ ID NO: 7; and a light chain variable region containing a light chain CDR1 containing an amino acid sequence of R-A-S-Q-X9-I-X10-X11-X12-L-N (SEQ ID NO: 8), a light chain CDR2 containing an amino acid sequence of A-X13-S-X14-L-Q-S (SEQ ID NO: 9), and a light chain CDR3 containing an amino acid sequence of Q-Q-X15-Y-X16-X17-P-X18-T (SEQ ID NO: 10).

Another aspect of the present invention provides a chimeric antigen receptor (CAR) containing: an extracellular binding domain containing an antigen binding site that specifically binds to PD-L1; a transmembrane domain; and an endodomain, wherein the antigen binding site that specifically binds to PD-L1 is a single-chain variable fragment (ScFv) of an anti-PD-L1 antibody including a heavy chain variable region containing a heavy chain CDR1 containing an amino acid sequence of X1-Y-X2-M-X3 (SEQ ID NO: 1), a heavy chain CDR2 containing an amino acid sequence of X₄-I-S-X₅-S-G-X₆-X₇-X_{B}-Y-Y-A-D-S-V-K-G (SEQ ID NO: 2), and a heavy chain CDR3 containing any one sequence selected from the group consisting of SEQ ID NO: 3, SEQ ID NO: 4, SEQ ID NO: 5, SEQ ID NO: 6, and SEQ ID NO: 7; and a light chain variable region containing a light chain CDR1 containing an amino acid sequence of R-A-S-Q-X₉-I-X₁₀-X₁₁-X₁₂-L-N (SEQ ID NO: 8), a light chain CDR2 containing an amino acid sequence of A-X₁₃-S-X₁₄-L-Q-S (SEQ ID NO: 9), and a light chain CDR3 containing an amino acid sequence of Q-Q-X₁₅-Y-X₁₆-X₁₇-P-X₁₈-T (SEQ ID NO: 10).

Still another aspect of the present invention provides a polynucleotide containing a base sequence encoding the chimeric antigen receptor and an expression vector containing the polynucleotide.

Still another aspect of the present invention provides an immune cell expressing the chimeric antigen receptor on a surface thereof.

Still another aspect of the present invention provides a pharmaceutical composition for treating or preventing cancer containing the immune cell.

### ADVANTAGEOUS EFFECTS

The antibody of the present invention, the antigen-binding fragment thereof, and the chimeric antigen receptor using the same may specifically bind to PD-L1 mainly expressed in cancer cells, and accordingly, signal transduction occurs in immune cells expressing the chimeric antigen receptor, the cytotoxicity or cytolytic activity of the immune cells is significantly improved and the secretion of cytokines is promoted. In addition, there is an effect of increasing degranulation of cancer cells co-cultured with the immune cells.

Therefore, the antibody of the present invention, the chimeric antigen receptor using the same, and the immune cell expressing the same may be usefully used for treating cancer.

However, the effects of the present invention are not limited to the effects mentioned above, and other effects that are not mentioned may be obviously understood by those skilled in the art from the following description.

### BRIEF DESCRIPTION OF THE DRAWINGS

FIG. 1 is a schematic diagram showing a structure of a chimeric antigen receptor (PD-L1 CAR) of the present invention containing an antigen-binding variable fragment (scFv) that specifically binds to PD-L1 as an extracellular binding domain.
FIG. 2 illustrates the results of confirming whether the chimeric antigen receptors of the present invention are expressed in natural killer cells ("PD-L1_E1 CAR NK cells", "PD-L1_C4 CAR NK cells", "PD-L1 E7 CAR NK cells", "PD-L1_E8 CAR NK cells", and "PD-L1_C8 CAR NK cells") into which five types of genes of chimeric antigen receptors of the present invention are introduced and expressed.
FIG. 3 illustrates the results of confirming the expression of PD-L1 in MDA-MB-231 cells, which is a breast cancer cell line.
FIG. 4 illustrates the results showing that all five types of PD-L1 CAR NK cells have significantly higher cytotoxicity against MDA-MB-231 cells expressing PD-L1 than a control NK cell that does not express PD-L1 CAR on a surface thereof.
FIG. 5A illustrates the results of comparing the amounts of cytokines (IFN-γ) secreted by natural killer cells when NK cells into which the chimeric antigen receptor PD-L1_E1 CAR of the present invention is introduced are co-cultured with MDA-MB-231 cells expressing PD-L1 and AU565 cells not expressing PD-L1.
FIG. 5B illustrates the results of comparing the expression levels of Cd107a, which is an indicator of degranulation, when NK cells into which the chimeric antigen receptor PD-L1_E1 CAR of the present invention is introduced are co-cultured with MDA-MB-231 cells expressing PD-L1 and AU565 cells not expressing PD-L1.
FIG. 6 illustrates a procedure from formation of immune synapses (stage A) to final death of cancer cells (stage E) in a process in which immune cells kill cancer cells by dividing the procedure into five stages including stage A to stage E.
FIG. 7A illustrates a degree of immune synapse formation progression and a time required to go through all the stages A to E when PD-L1_E1 CAR NK cells of the present invention and PURO-92 cells that do not express PD-L1 are treated on MDA-MB-231 cell line. FIG. 7B illustrates the results showing a rate of cell death relative to contact when PD-L1_E1 CAR NK cells of the present invention and PURO-92 cells that do not express PD-L1 are brought into contact with MDA-MB-231 cell line.
FIG. 8 illustrates the results of confirming the expression of PD-L1 in H460 cells, which is a lung cancer cell line.
FIG. 9 illustrates the result that when H460 cells expressing PD-L1 are treated with PD-L1_E1 CAR NK cells at E:T ratios of 4:1, 2:1, and 1:1, all the cells have much higher cytotoxicity compared to a control NH cell that does not express PD-L1 CAR on a surface thereof.
FIG. 10 illustrates an administration schedule of cancer cell lines and NK cells to mice.
FIG. 11 illustrates the results of measuring tumor sizes based on the time of administration of cancer cells to mice after administering cancer cell lines, PD-L1_E1 CAR NK cells of the present invention, and dEcto CAR NK cells that do not express PD-L1 CAR.
FIGS. 12A and 12B are a photograph and a graph showing the results of measuring weights of tumors after a predetermined period of time has elapsed after administration of cancer cell line, PD-L1_E1 CAR NK cells of the present invention, and the dEcto CAR NK cells that do not express PD-L1 CAR to mice.

### BEST MODE FOR CARRYING OUT THE INVENTION

Hereinafter, the present invention will be described in detail.

### 1. Novel anti-PD-L1 antibody and antigen-binding fragment thereof

An aspect of the present invention provides an anti-PD-L1 antibody and an antigen-binding fragment thereof that may specifically bind to PD-L1.

The antibody of the present invention or the antigen-binding fragment thereof includes a heavy chain variable region and a light chain variable region.

The heavy chain variable region included in the antibody of the present invention or the antigen-binding fragment thereof may contain CDR1 containing an amino acid sequence of X1-Y-X2-M-X3 (SEQ ID NO: 1), and X₁ to X₃ in the amino acid sequence of SEQ ID NO: 1 may each independently be any amino acid.

In the amino acid sequence of SEQ ID NO: 1, X₁ may be any one selected from the group consisting of aspartic acid, glutamic acid, serine, threonine, asparagine, and glutamine, or any amino acid that may be conservatively substituted therewith. More specifically, X₁ may be any one selected from the group consisting of aspartic acid, serine, and asparagine.

In the amino acid sequence of SEQ ID NO: 1, X₂ may be any one selected from the group consisting of alanine, leucine, isoleucine, glycine, aspartic acid, and glutamic acid, or any amino acid that may be conservatively substituted therewith. More specifically, X₂ may be any one selected from the group consisting of alanine, glycine, and glutamic acid.

In the amino acid sequence of SEQ ID NO: 1, X₃ may be any one selected from the group consisting of serine, threonine, asparagine, glutamine, histidine, arginine, and lysine, or any amino acid that may be conservatively substituted therewith. More specifically, X₃ may be any one selected from the group consisting of serine, asparagine, and histidine.

The amino acid sequence of SEQ ID NO: 1 may be any one selected from the group consisting of amino acid sequences of D-Y-E-M-S (SEQ ID NO: 11), D-Y-A-M-S (SEQ ID NO: 16), S-Y-AM-N (SEQ ID NO: 21), N-Y-G-M-H (SEQ ID NO: 26), and D-Y-A-M-H (SEQ ID NO: 31), or an amino acid sequence having 70% or more, 80% or more, 90% or more, 95% or more, or 99% or more homology with the amino acid sequence. More specifically, the amino acid sequence of SEQ ID NO: 1 may include an amino acid sequence of D-Y-E-M-S (SEQ ID NO: 11).

The heavy chain variable region included in the antibody of the present invention or the antigen-binding fragment thereof may further contain a CDR2 containing an amino acid sequence of X₄-I-S-X₅-S-G-X₆-X₇-X₈-Y-Y-A-D-S-V-K-G (SEQ ID NO: 2), and in the amino acid sequence of SEQ ID NO: 2, X₄ to X₈ may each independently be any amino acid.

In the amino acid sequence of SEQ ID NO: 2, X₄ may be any one selected from the group consisting of alanine, leucine, isoleucine, arginine, histidine, lysine, glycine, alanine, leucine, and isoleucine, or any amino acid that may be conservatively substituted therewith. More specifically, X₄ may be any one selected from the group consisting of alanine, arginine, and glycine.

In the amino acid sequence of SEQ ID NO: 2, X₅ may be any one selected from the group consisting of serine, threonine, glutamine, asparagine, glycine, alanine, leucine, and isoleucine, or any amino acid that may be conservatively substituted therewith. More specifically, X₅ may be any one selected from the group consisting of serine, glutamine, and glycine.

In the amino acid sequence of SEQ ID NO: 2, X₆ may be any one selected from the group consisting of glycine, alanine, leucine, isoleucine, serine, and threonine, or any amino acid that may be conservatively substituted therewith. More specifically, X₆ may be any one selected from the group consisting of glycine and serine.

In the amino acid sequence of SEQ ID NO: 2, X₇ may be any one selected from the group consisting of threonine, serine, tyrosine, phenylalanine, tryptophan, serine, threonine, arginine, histidine, and lysine, or any amino acid that may be conservatively substituted therewith. More specifically, X₇ may be any one selected from the group consisting of threonine, tyrosine, serine, and arginine.

In the amino acid sequence of SEQ ID NO: 2, X₈ may be any one selected from the group consisting of alanine, leucine, isoleucine, serine, threonine, arginine, histidine, and lysine, or any amino acid that may be conservatively substituted therewith. More specifically, X₈ may be any one selected from the group consisting of isoleucine, threonine, and lysine.

The amino acid sequence of SEQ ID NO: 2 may be any one selected from the group consisting of amino acid sequences of A-I-S-G-S-G-S-Y-T-Y-Y-A-D-S-V-K-G (SEQ ID NO: 12), A-I-S-Q-S-G-G-T-I-Y-Y-A-D-S-V-K-G (SEQ ID NO: 17), R-I-S-S-S-G-G-Y-T-Y-Y-A-D-S-V-K-G (SEQ ID NO: 22), A-I-S-S-S-G-G-S-T-Y-Y-A-D-S-V-K-G (SEQ ID NO: 27), and G-I-S-S-S-G-S-R-K-Y-Y-A-D-S-V-K-G (SEQ ID NO: 32), or an amino acid sequence having 70% or more, 80% or more, 90% or more, 95% or more, or 99% or more homology with the amino acid sequence. More specifically, the amino acid sequence of SEQ ID NO: 2 may include an amino acid sequence of A-I-S-G-S-G-S-Y-T-Y-Y-A-D-S-V-K-G (SEQ ID NO: 12).

The heavy chain variable region included in the antibody of the present invention or the antigen-binding fragment thereof may further contain a CDR3, which is any one selected from the group consisting of G-R-G-R-V-L-W-T-T-F-D-H (SEQ ID NO: 3), S-T-V-S-S-L-Q-R-G-F-D-L (SEQ ID NO: 4), R-R-T-L-V-S-A-F-D-V (SEQ ID NO: 5), R-G-S-S-R-G-A-F-D-Y (SEQ ID NO: 6), and D-R-I-W-Y-Q-G-S-G-F-D-I (SEQ ID NO: 7), or contains an amino acid sequence having 70% or more, 80% or more, 90% or more, 95% or more, or 99% or more homology with the amino acid sequence. More specifically, the CDR3 may contain an amino acid sequence of D-R-I-W-Y-Q-G-S-G-F-D-I (SEQ ID NO: 7).

In addition, the light chain variable region included in the antibody of the present invention or the antigen-binding fragment thereof contains a CDR1 containing an amino acid sequence of R-A-S-Q-X₉-I-X₁₀-X₁₁-X₁₂-L-N (SEQ ID NO: 8), and in the amino acid sequence of SEQ ID NO: 8, X₉ to X₁₂ may each independently be any amino acid.

In the amino acid sequence of SEQ ID NO: 8, X₉ may be any one selected from the group consisting of aspartic acid, glutamic acid, serine, and threonine, or any amino acid that may be conservatively substituted therewith. More specifically, X₉ may be any one selected from the group consisting of aspartic acid and serine.

In the amino acid sequence of SEQ ID NO: 8, X₁₀ may be any one selected from the group consisting of serine, threonine, glycine, alanine, leucine, and isoleucine, or any amino acid that may be conservatively substituted therewith. More specifically, X₁₀ may be any one selected from the group consisting of serine and glycine.

In the amino acid sequence of SEQ ID NO: 8, X₁₁ may be any one selected from the group consisting of asparagine, glutamine, serine, and threonine, or any amino acid that may be conservatively substituted therewith. More specifically, X₁₁ may be any one selected from the group consisting of asparagine and serine.

In the amino acid sequence of SEQ ID NO: 8, X₁₂ may be any one selected from the group consisting of tryptophan, tyrosine, and phenylalanine, or any amino acid that may be conservatively substituted therewith. More specifically, X₁₂ may be any one selected from the group consisting of tryptophan and tyrosine.

The amino acid sequence of SEQ ID NO: 8 may be any one selected from the group consisting of amino acid sequences of R-A-S-Q-S-I-S-S-W-L-N (SEQ ID NO: 13), R-A-S-Q-D-I-S-N-W-L-N (SEQ ID NO: 18), R-A-S-Q-S-I-S-N-Y-L-N (SEQ ID NO: 23), R-AS-Q-S-I-S-N-W-L-N (SEQ ID NO: 28), and R-A-S-Q-D-I-G-N-Y-L-N (SEQ ID NO: 33), or an amino acid sequence having 70% or more, 80% or more, 90% or more, 95% or more, or 99% or more homology with the amino acid sequence. More specifically, the amino acid sequence of SEQ ID NO: 8 may include an amino acid sequence of R-A-S-Q-S-I-S-S-W-L-N (SEQ ID NO: 13).

The light chain variable region included in the antibody of the present invention or the antigen-binding fragment thereof contains a light chain CDR2 containing an amino acid sequence of A-X₁₃-S-X₁₄-L-Q-S (SEQ ID NO: 9), and in the amino acid sequence of SEQ ID NO: 9, X₁₃ and X₁₄ may each independently be any amino acid.

In the amino acid sequence of SEQ ID NO: 9, X₁₃ may be any one selected from the group consisting of serine, threonine, glycine, alanine, leucine, and isoleucine, or any amino acid that may be conservatively substituted therewith. More specifically, X₁₃ may be any one selected from the group consisting of threonine and alanine.

The amino acid sequence of SEQ ID NO: 9 may be any one selected from the group consisting of amino acid sequences of A-A-S-N-L-Q-S (SEQ ID NO: 14), A-T-S-R-L-Q-S (SEQ ID NO: 19), A-T-S-S-L-Q-S (SEQ ID NO: 24), A-A-S-S-L-Q-S (SEQ ID NO: 29), and A-A-S-R-L-Q-S (SEQ ID NO: 34), or an amino acid sequence having 70% or more, 80% or more, 90% or more, 95% or more, or 99% or more homology with the amino acid sequence. More specifically, the amino acid sequence of SEQ ID NO: 9 may include an amino acid sequence of A-A-S-N-L-Q-S (SEQ ID NO: 14) .

The light chain variable region included in the antibody of the present invention or the antigen-binding fragment thereof contains a light chain CDR3 containing an amino acid sequence of Q-Q-X₁₅-Y-X₁₆-X₁₇-P-X₁₈-T (SEQ ID NO: 10), and in the amino acid sequence of SEQ ID NO: 10, X₁₅ to X₁₈ may each independently be any amino acid.

In the amino acid sequence of SEQ ID NO: 10, X₁₅ and X₁₆ may each independently be any one selected from the group consisting of serine and threonine, or any amino acid that may be conservatively substituted therewith.

In the amino acid sequence of SEQ ID NO: 10, X₁₇ may be any one selected from the group consisting of serine, threonine, phenylalanine, tryptophan, and tyrosine, or any amino acid that may be conservatively substituted therewith. More specifically, X₁₇ may be any one selected from the group consisting of threonine and phenylalanine.

In the amino acid sequence of SEQ ID NO: 10, X₁₈ may be any one selected from the group consisting of leucine, isoleucine, phenylalanine, tryptophan, and tyrosine, or any amino acid that may be conservatively substituted therewith. More specifically, X₁₈ may be any one selected from the group consisting of leucine, tryptophan, and tyrosine.

The amino acid sequence of SEQ ID NO: 10 may be any one selected from the group consisting of amino acid sequences of Q-Q-S-Y-S-F-P-W-T (SEQ ID NO: 15), Q-Q-T-Y-S-T-P-L-T (SEQ ID NO: 20), Q-Q-S-Y-T-F-P-W-T (SEQ ID NO: 25), Q-Q-S-Y-S-F-P-W-T (SEQ ID NO: 30), and Q-Q-S-Y-S-T-P-Y-T (SEQ ID NO: 35), or an amino acid sequence having 70% or more, 80% or more, 90% or more, 95% or more, or 99% or more homology with the amino acid sequence. More specifically, the amino acid sequence of SEQ ID NO: 10 may include an amino acid sequence of Q-Q-S-Y-S-F-P-W-T (SEQ ID NO: 15).

The amino acid sequences described above may include variants containing different sequences due to deletion, insertion, or substitution of an amino acid residue, or a combination thereof within a range that does not affect a structure, function, activity, and the like of the polypeptide containing the same. In addition, the amino acid sequences may include amino acids that have undergone common modifications known in the art, and examples of the modification of the amino acid include phosphorylation, sulfation, acrylation, glycosylation, methylation, and famesylation. The humanized antibody of the present invention or the antigen-binding fragment thereof not only contains the amino acid sequences described above, but also contains an amino acid sequence that is substantially identical thereto or a variant thereof. The meaning of having the substantially identical amino acid sequence is that an amino acid sequence has 90% or more, 91% or more, 92% or more, 93% or more, 94% or more, 95% or more, 96% or more, 97% or more, 98% or more, 99% or more, or 99.5% or more homology with the amino acid sequence described above, but is not limited thereto.

### 2. Chimeric antigen receptor (CAR) containing novel antibody or antigen-binding fragment thereof and polynucleotide and expression vector for expressing the same

An aspect of the present invention provides an anti-PD-L1 antibody and an antigen-binding fragment thereof that may specifically bind to PD-L1.

The term "antibody" in the present invention refers to an immunoglobulin molecule having immunological responsiveness by specifically binding to an epitope of an antigen. The antibody may include all of a monoclonal antibody, a polyclonal antibody, an antibody having a full-length chain structure (a full-length antibody), a functional fragment (antigen-binding fragment) having at least an antigen binding function, and a recombinant antibody, and specifically, the antibody of the present invention may be a monoclonal antibody or an antigen-binding fragment thereof. The monoclonal antibody refers to an antibody molecule of a single molecular composition obtained from a substantially identical antibody population, and the monoclonal antibody exhibits single binding specificity and affinity for a specific epitope. The full-length antibody has a structure having two full-length light chains and two full-length heavy chains, and each light chain may be linked to the heavy chain through a disulfide bond. The antibody may contain heavy chain (HC) and light chain (LC) polypeptides, and the heavy chain and the light chain may include a variable region and a constant region.

The constant region is a site that mediates the binding of the antibody to various types of cells of an immune system, host tissue containing components of a complement system, and the like. The constant region has the same function regardless of the type of antigen as long as it is the same type of antibody derived from the same species, and the amino acid sequence constituting the constant region is identical or highly similar for each antibody. The constant region may be divided into a heavy chain constant region (may be abbreviated as CH) and a light chain constant region (may be abbreviated as CL). The heavy chain constant region has gamma (γ), mu (µ), alpha (α), delta (δ) , and/or epsilon (ε) type, and has gamma 1 (γ1), gamma 2 (γ2), gamma 3 (γ3), gamma 4 (γ4), alpha 1 (α1), and/or alpha 2 (α2) as a subclass. The light chain constant region has kappa (κ) and lambda (λ) types. IgG includes IgG1, IgG2, IgG3, and IgG4 as a subtype.

The variable region is an antibody site that has specificity for an antigen, and may be divided into a heavy chain variable region (may be abbreviated as VH) and a light chain variable region (may be abbreviated as VL). The variable region may include three complementary-determining regions (CDRs) and four framework regions (FRs). The CDR may be a ring-shaped site involved in recognition of an antigen, and specificity for the antigen may be determined according to an amino acid sequence of the CDR. The CDRs may be referred to as CDR1, CDR2, and CDR3 according to the order thereof, and may be referred to as CDR-H1, CDR-H2, and CDR-H3 for the heavy chain variable regions and CDR-L1, CDR-L2, and CDR-L3 for the light chain variable regions, depending on whether the CDR is of a heavy chain or light chain polypeptide. Similarly, the FRs may be referred to as FR-H1, FR-H2, FR-H3, and FR-H4 for the heavy chain variable regions, and may be referred to as FR-L1, FR-L2, FR-L3, and FR-L4 for the light chain variable regions. In addition, the CDRs and the FRs may be arranged in the following order in each variable region.

The term "antigen-binding fragment" in the present invention refers to any fragment of the humanized antibody of the present invention that retains the antigen-binding function of the antibody. The antigen-binding fragment may be referred to interchangeably with terms such as "fragment" and "antibody fragment", and the antigen-binding fragment may be Fab, Fab', F(ab')₂, Fv, or the like, but is not limited thereto.

The Fab has a structure having variable regions of a light chain and a heavy chain, a constant region of the light chain, and a first constant region of the heavy chain (CH1 domain), and has one antigen binding site. The Fab' differs from the Fab in that the Fab' has a hinge region containing one or more cysteine residues at the C-terminus of the heavy chain CH1 domain. The F(ab')₂ is generated when the cysteine residue of the hinge region of the Fab' forms a disulfide bond. The Fv refers to the minimum antibody fragment with only a heavy chain variable region and a light chain variable region. The two-chain Fv may be formed by linking the heavy chain variable region and the light chain variable region through a non-covalent bond, and the single-chain Fv may be formed by linking the heavy chain variable region and the light chain variable region via a peptide linker through a covalent bond or directly linking the heavy chain variable region and the light chain variable region at the C-terminus, thereby forming a structure such as a dimer like the two-chain Fv. The antigen-binding fragment may be prepared by using a protease (for example, the Fab may be obtained by restriction cleavage of the entire antibody with papain, and the F(ab')2 fragment may be obtained by cleavage with pepsin), or through a genetic recombination technology, but is not limited thereto.

The linker may be a peptide linker and may have a length of about 10 to 25 amino acids. For example, the linker may contain a hydrophilic amino acid such as glycine (G) and/or serine (S). The linker may contain, for example, (GS)n, (GGS)n, (GSGGS) n, or (GnS) m (n and m are each 1 to 10), and may contain, for example, (GnS)m (n and m are each 1 to 10), but is not limited thereto.

The term "epitope" in the present invention refers to a specific site on an antigen that may be specifically recognized and bound by an immunoglobulin, an antibody, or an antigen-binding fragment thereof. The epitope may be formed from contiguous amino acids or from noncontiguous amino acids juxtaposed by a tertiary folding of a protein.

The "specifically binding" may mean binding to other molecules with a binding affinity greater than the background binding, for example, the extracellular binding domain may bind to a target antigen with an affinity of about 10⁻⁵ M or more or with Ka (an equilibrium dissociation constant of a specific binding interaction with a unit of 1/M). Regarding the affinity, an equilibrium dissociation constant (Kd) of a specific binding interaction with a unit of M may be in a range of 10⁻⁵ M to 10⁻¹³ M or may be equal to or less than the range.

The antibody of the present invention or the antigen-binding fragment thereof may further include, for example, a heavy chain constant region and/or a light chain constant region of a human-derived antibody, and the heavy chain constant region and/or the light chain constant region of the human-derived antibody may be used without limitation in type or amino acid sequence, as long as the antibody or the antigen-binding fragment thereof does not inhibit the property of specifically binding to PD-L1.

The term "chimeric antigen receptor (CAR)" in the present invention refers to a synthetic complex designed to induce an immune response against a target antigen and a cell expressing the antigen when the target antigen and the cell expressing the antigen are recognized and bound. The chimeric antigen receptor may contain an extracellular binding domain, a transmembrane domain, and an intracellular signaling domain. The chimeric antigen receptor is expressed on the surface of the immune cell, and recognizes and binds to a specific antigen, for example, an antigen specifically expressed on a surface of a cancer cell, through an antigen binding site included in the extracellular binding domain, thereby causing signal transduction in the immune cell and changing the activity of the immune cell, and thus inducing an immune response targeting only the specific antigen.

The chimeric antigen receptor (CAR) of the present invention contains: an extracellular binding domain containing an antigen binding site that specifically binds to PD-L1; a transmembrane domain; and an intracellular signaling domain.

In a case where the chimeric antigen receptor of the present invention is expressed on the surface of the immune cell, when PD-L1, which is a target antigen, binds to the receptor, signal transduction occurs within the immune cell, and the improvement of cytotoxicity (or cytolytic activity) of the immune cell and/or the promotion of cytokine secretion of the immune cell may be induced. The improvement of cytotoxicity (or cytolytic activity) or the promotion of cytokine secretion may mean exhibiting a higher level of cytotoxicity (or cytolytic activity) or cytokine secretion than a level of cytotoxicity (or cytolytic activity) or cytokine secretion exhibited by an immune cell in which an antigen is not present.

The extracellular binding domain may further include at least one selected from the group consisting of a hinge domain and a spacer domain. The antigen binding site of the extracellular binding domain may be linked to the transmembrane domain through the hinge domain and/or the spacer domain.

The hinge domain is a part that enables the antigen binding site to be physically spaced apart from the surface of the immune cell on which the chimeric antigen receptor is expressed, so as to allow appropriate cell/cell contact, binding of an appropriate antigen/antigen binding site, and activation of an appropriate chimeric antigen receptor, and may play an important role in determining the position of the extracellular binding domain. The chimeric antigen receptor may contain one or more hinge domains between the extracellular binding domain and the transmembrane domain. The hinge domain may be derived from a natural, synthetic, semi-synthetic, or recombinant source. The hinge domain may contain an amino acid sequence of a naturally occurring immunoglobulin hinge region or a modified immunoglobulin hinge region. The modified hinge region refers to (a) a naturally occurring hinge region having an amino acid modification of up to 30% (for example: amino acid substitution or deletion of up to 25%, 20%, 15%, 10%, or 5%), (b) a part of the naturally occurring hinge region of at least 10 amino acids (for example: at least 12, 13, 14, or 15 amino acids) in length, which has an amino acid modification of up to 30% (for example: amino acid substitution or deletion of up to 25%, 20%, 15%, 10%, or 5%), or (c) a part of the naturally occurring hinge region including a core hinge region (which may be 4, 5, 6, 7, 8, 9, 10, 11, 12, 13, 14, or 15, or at least 4, 5, 6, 7, 8, 9, 10, 11, 12, 13, 14, or 15 amino acids in length). In a certain embodiment, one or more cysteine residues in the naturally occurring immunoglobulin hinge region may be substituted with one or more other amino acid residues (for example, one or more serine residues). The modified immunoglobulin hinge region may alternatively or additionally have another amino acid residue such as a proline residue of a wild-type immunoglobulin hinge region substituted with cysteine. The hinge domain may be a hinge region derived from an extracellular binding domain of a type 1 membrane protein such as CD8, CD4, CD28, or CD7, but any hinge domain may be used without limitation as long as the antigen binding site, the transmembrane domain, and the intracellular signaling domain may be linked via the cell membrane. In addition, the hinge domain may be a wild-type hinge region from these molecules or may be modified.

The spacer domain may be referred to as a linking domain, may include, for example, a hinge domain derived from CD28 and/or a hinge domain derived from CD8, and may include all or a part of the hinge domain derived from CD28 and/or the hinge domain derived from CD8.

The hinge domain and/or the spacer domain may be at least one selected from the group consisting of a Myc epitope, a CD8 hinge domain, and an Fc, and specifically, may include a Myc epitope and a CD8 hinge domain. More specifically, the Myc epitope may contain an amino acid sequence of SEQ ID NO: 17, and the CD8 hinge domain may contain an amino acid sequence of SEQ ID NO: 18 or SEQ ID NO: 19.

The transmembrane domain refers to a partial region that serves to link and fuse the extracellular binding domain and the intracellular signaling domain to each other and to fix the chimeric antigen receptor to a plasma membrane of the immune cell. The transmembrane domain may be derived from a natural, synthetic, semi-synthetic, or recombinant source. The transmembrane domain may be any one selected from the group consisting of an alpha (α), beta (β), or zeta (ζ) chain of a T-cell receptor (TCR), CD28, CD3 epsilon (ε), CD45, CD4, CD5, CD8, CD9, CD16, CD22, CD33, CD37, CD64, CD80, CD86, CD134, CD137, and CD154, but is not limited thereto.

The transmembrane domain may be attached to the extracellular binding domain via a linker. For example, the linker may be a short oligopeptide or polypeptide linker having a length of 2 to 10 amino acids, for example, a glycine (G)-serine (S) doublet, but is not limited thereto.

The intracellular signaling domain corresponds to a portion that serves to transmit a signal generated according to the binding of the chimeric antigen receptor and the antigen into the immune cell in order to induce the function of the immune cell (for example, activation including the release of cytotoxicity (or cytolytic activity) factors to the target cell in which the chimeric antigen receptor and antigen are bound, cytokine production, proliferation and cytotoxicity or cytolytic activity, or other cellular responses induced by the antigen binding). The intracellular signaling domain may be a part of a protein that transmits an effector function signal and directs the cell to perform a special function.

As the intracellular signaling domain, an intracellular signaling domain which has been used in the development process of the existing chimeric antigen receptor may be used. Specifically, the intracellular signaling domain may contain only CD3ζ used in the first-generation chimeric antigen receptor (CAR). In addition, as used in the second-generation CAR, the form in which a co-stimulatory domain (CD28 or CD137/4-1BB) and CD3ζ are bound may be used to improve the responsiveness to immune cells. In addition, two or more co-stimulatory domains may be used as used in the third-generation CAR, and in this case, the co-stimulatory domains may be bound to 4-1BB, CD28, or OX40, and the like, to achieve extension and persistence of an immune cell containing the CAR in vivo. Further, as used in the fourth-generation CAR, an additional gene encoding a cytokine such as IL-12 or IL-15 may be contained so that a CAR-based immune protein of the cytokine may be further expressed, and as used in the fifth-generation CAR, an interleukin receptor chain, for example, IL-2Rβ may be further contained to enhance the immune cell.

When the chimeric antigen receptor of the present invention is expressed on the surface of the immune cell, and recognizes and binds to PD-L1, the cytotoxicity or cytolytic activity of the immune cell may be improved, or the secretion of cytokines by the immune cell may be induced. Therefore, when the antigen binding site of the chimeric antigen receptor recognizes and binds to an antigen when a cancer cell expressing PD-L1 is present, the cytotoxicity or cytolytic activity of the immune cell and/or cytokine secretion may be induced through signal transduction, and thus, the chimeric antigen receptor may be usefully used as a chimeric antigen receptor having excellent cytotoxicity or cytolytic efficacy for attacking cancer cells.

Another aspect of the present invention provides a polynucleotide and an expression vector for expressing the chimeric antigen receptor.

The polynucleotide contains a base sequence encoding the chimeric antigen receptor.

The term "polynucleotide" in the present invention comprehensively includes DNA (gDNA and cDNA) and RNA molecules, and the nucleotide, which is a basic structural unit, includes not only a natural nucleotide but also an analogue having a sugar or a base site modified.

Encoding the chimeric antigen receptor means that the polynucleotide encodes genetic information that may synthesize a protein containing the amino acid sequence of the chimeric antigen receptor of the present invention through a typical protein expression process such as transcription and translation. In this case, the scope of the present invention may include even a polynucleotide that encodes not only a protein containing an amino acid sequence completely identical to that of the chimeric antigen receptor, but also a protein containing an amino acid sequence substantially identical to that of the protein as described above or a protein having the identical and/or similar activity to that of the protein.

Specifically, the polynucleotide of the present invention may contain a base sequence encoding an antigen-binding variable fragment of the anti-PD-L1 antibody that specifically binds to PD-L1, and more specifically, may contain a base sequence of SEQ ID NO: 11, SEQ ID NO: 12, SEQ ID NO: 7, SEQ ID NO: 13, SEQ ID NO: 14, or SEQ ID NO: 15.

In addition, the polynucleotide of the present invention may contain a base sequence encoding the antigen-binding variable fragment as well as a base sequence encoding another extracellular binding domain portion linked thereto, the transmembrane domain, and/or the intracellular signaling domain.

The descriptions of the chimeric antigen receptor, including the antibody, the antigen-binding variable fragment, the extracellular binding domain, the transmembrane domain, and the intracellular signaling domain, are the same as those described above.

The polynucleotide of the present invention may contain a base sequence substantially identical to the base sequences listed above. The substantially identical base sequence includes, for example, a case in which the same amino acid may be synthesized when transcribed and translated, and may be a base sequence having 90% or more, 91% or more, 92% or more, 93% or more, 94% or more, 95% or more, 96% or more, 97% or more, 98% or more, 99% or more, or 99.5% or more homology with the base sequences listed above, but is not limited thereto.

The polynucleotide encoding the chimeric antigen receptor may contain an optimized base sequence depending on the type of organism into which the polynucleotide is introduced and expressed, and an expression system such as transcription or translation of the organism. This is due to degeneracy of a codon, and accordingly, various combinations of nucleotide sequences that may encode the protein expressed thereby may exist, all of which fall within the scope of the present invention. The modification of the polynucleotide according to the codon optimization may be determined according to the type of organism in which the chimeric antigen receptor of the present invention is expressed and applied, and for example, the polynucleotide of the present invention may be a polynucleotide modified by being optimized for selection of a codon of a mammal or a primate, and more specifically, may be modified by being optimized to be suitable for the expression and action from a human.

The expression vector of the present invention contains the polynucleotide.

Since the polynucleotide contains a base sequence encoding the chimeric antigen receptor of the present invention, the expression vector containing the polynucleotide may be used to express and produce the chimeric antigen receptor, and may serve to transfer the polynucleotide to a specific cell or organism or may be used to keep and store the polynucleotide so that the chimeric antigen receptor may be expressed.

The expression vector may be constructed using a prokaryotic cell or a eukaryotic cell as a host.

For example, when the expression vector uses a prokaryotic cell as a host, the expression vector generally contains a strong promoter capable of advancing transcription (for example, tac promoter, lac promoter, lacUV5 promoter, lpp promoter, pLλ, promoter, pRλ promoter, rac5 promoter, amp promoter, recA promoter, SP6 promoter, trp promoter, T7 promoter, or the like), a ribosome binding site for initiating translation, and a transcription/translation termination sequence. When *E. coli* (for example, HB101, BL21, DH5α, or the like) is used as a host cell, a promoter and operator site of an *E. coli* tryptophan biosynthesis pathway (Yanofsky, C, J Bacteriol, (1984) 158:1018-1024) and a leftward promoter of phage λ (pLλ promoter; Herskowitz, I and Hagen, D, Ann Rev Genet, (1980) 14:399-445) may be used as regulatory sites. When *Bacillus* bacteria are used as a host cell, a promoter of toxin protein gene of *Bacillus thuringiensis* (Appl Environ Microbiol (1998) 64:3932-3938; Mol Gen Genet (1996) 250:734-741) or any promoter that may be expressed in *Bacillus* bacteria may be used as a regulatory site. The expression vector may be produced by manipulating a plasmid (for example, pCL, pSC101, pGV1106, pACYC177, ColE1, pKT230, pME290, pBR322, pUC8/9, pUC6, pBD9, pHC79, pIJ61, pLAFR1, pHV14, pGEX series, pET series, pUC19, or the like), a phage (for example, λgt4·λB, λ-Charon, λΔz1, M13, or the like), or a virus (for example, SV40 or the like), which is often used in the art.

In a case where the expression vector uses the eukaryotic cell as a host, a promoter derived from a genome of a mammalian cell (for example, metallothionine promoter, β-actin promoter, human hemoglobin promoter, or human muscle creatine promoter) or a promoter derived from a mammalian virus (for example, adenovirus late promoter, vaccinia virus 75K promoter, SV40 promoter, cytomegalovirus (CMV) promoter, tk promoter of HSV, mouse mammary tumor virus (MMTV) promoter, LTR promoter of HIV, promoter of Moloney virus, promoter of Epstein-Barr virus (EBV), and promoter of Roux Sarcoma virus (RSV)) may be used, and the expression vector may generally contain a polyadenylation sequence as a transcription termination sequence. The expression vector may contain a CMV promoter.

In addition, the expression vector may be fused with other sequences to facilitate purification of the antibody expressed therefrom. Examples of the sequences to be fused include glutathione S-transferase (Pharmacia, USA), maltose binding protein (NEB, USA), FLAG (IBI, USA), and 6x His (hexahistidine; Quiagen, USA). In addition, since the protein expressed by the expression vector of the present invention is a chimeric antigen receptor, the expressed protein may be easily purified through a protein A column or the like without an additional sequence for purification in consideration of characteristics thereof.

The expression vector contains an antibiotic resistance gene conventionally used in the art as a selectable marker, and may contain, for example, resistance genes against ampicillin, gentamicin, carbenicillin, chloramphenicol, streptomycin, kanamycin, geneticin, neomycin, and tetracycline.

### 3. Immune cell expressing chimeric antigen receptor for PD-L1 on surface thereof

Another aspect of the present invention provides an immune cell that exhibits improved cytotoxicity or cytolytic activity against a cancer cell expressing PD-L1.

The immune cell expresses the chimeric antigen receptor of the present invention described above on a surface thereof.

The chimeric antigen receptor is the same as that described in "2. Chimeric antigen receptor (CAR) containing novel antibody or antigen-binding fragment thereof and polynucleotide and expression vector for expressing the same". Specifically, the chimeric antigen receptor may contain an antigen binding site capable of specifically binding to a PD-L1 antigen expressed in a cancer cell.

The immune cell may be used without limitation as long as it is a cell that may induce immunity and cause a desired therapeutic effect, and may be, for example, any one selected from the group consisting of a natural killer cell (NK cell), a T cell, a natural killer T cell (NKT cell), a cytokine-induced killer cell (CIK), a macrophage, and a dendritic cell, but is not limited thereto. Therefore, the immune cell expressing the chimeric antigen receptor according to the present invention on a surface thereof may be a chimeric antigen receptor natural killer cell (CAR-NK cell), a chimeric antigen receptor T cell (CAR-T cell), a chimeric antigen receptor natural killer T cell (CAR-NKT cell), a chimeric antigen receptor macrophage (CAR-macrophage), or the like.

The T-cell may be, but is not limited to, a cytotoxic T lymphocyte (CTL), a tumor infiltrating lymphocyte (TIL), a T cell isolated from a peripheral blood mononuclear cell (PBMC), or the like.

The CAR-NK cell refers to a cell in which the chimeric antigen receptor is introduced into a natural killer cell, and has the advantage of being available as a general-purpose therapeutic agent by being able to target various cancer cells as well as solving, through on/off switching of the response, a problem due to persistent toxicity of a cancer immunotherapy when the existing CAR-T therapeutic agent based on the T cell is used, a risk of autoimmune diseases, a problem of the graft-versus-host disease (GVHD) in xenogeneic cell transplantation, a problem of off-target toxicity, and the like.

The immune cell of the present invention expresses a chimeric antigen receptor on a cell surface, the chimeric antigen receptor containing, as an extracellular binding domain, an antigen-binding variable fragment (scFv) of an antibody capable of specifically recognizing and binding to PD-L1 that may be specifically expressed in a cancer cell. Therefore, when the cancer cell expressing PD-L1 is present, signal transduction may occur through the chimeric antigen receptor, thereby further improving the cytotoxicity or cytolytic activity of immune cells and increasing the amount of cytokines secreted. Thus, the immune cell of the present invention may have the activity of attacking and treating cancer cells.

### 4. Use of immune cell of the present invention for treating cancer

Still another aspect of the present invention provides a pharmaceutical composition for treating cancer containing the immune cell.

Since the descriptions of the chimeric antigen receptor and the like are the same as those described in "2. Chimeric antigen receptor (CAR) containing novel antibody or antigen-binding fragment thereof and polynucleotide and expression vector for expressing the same", the description therefor is omitted to avoid repeated description.

The term "cancer" in the present invention is used in the same sense as "tumor", and refers to or means a physiological condition of a mammal, typically characterized by unregulated cell growth/proliferation.

Cancer or carcinoma that may be treated by the composition of the present invention is not particularly limited, and includes both solid cancer and blood cancer. For example, the cancer may be at least one selected from the group consisting of lung cancer, stomach cancer, ovarian cancer, cervical cancer, breast cancer, pancreatic cancer, colon cancer, colorectal cancer, esophageal cancer, skin cancer, thyroid cancer, kidney cancer, liver cancer, head and neck cancer, bladder cancer, prostate cancer, blood cancer, multiple myeloma, acute myeloid leukemia, malignant lymphoma, thymus cancer, osteosarcoma, fibrous tumor, and brain cancer, but is not limited thereto, and any cancer cell containing an antigen that may be recognized by the chimeric antigen receptor may be applied in the present invention without limitation.

The cancer may express PD-L1 on a surface of a cancer cell.

The term "treatment" in the present invention refers to inhibition of development of cancer and alleviation or elimination of symptoms.

The pharmaceutical composition may contain the immune cell in an amount of 1 to 10 times, 2 to 10 times, or 5 to 8 times greater than the number of tumor cells of an individual to be treated, but is not limited thereto.

In addition to the pharmaceutical composition, the composition may be in the form of a quasi-drug composition, a health food composition, or the like.

The composition for treating cancer of the present invention may further contain a pharmaceutically acceptable carrier. The "pharmaceutically acceptable" means that a substance does not inhibit activity of an active ingredient and does not have toxicity beyond what an application (prescription) target is adaptable to, and the "carrier" is defined as a compound that facilitates addition of a compound into cells or tissue.

The pharmaceutical composition of the present invention may be administered alone or in combination with any convenient carrier and the like, and a formulation to be administered may be a single dosage or repeated dosage form. The pharmaceutical composition may be a solid formulation or a liquid formulation. The solid formulation includes, but is not limited to, a powder, a granule, a tablet, a capsule, a suppository, and the like. The solid formulation may include, but is not limited to, a carrier, a flavoring agent, a binder, a preservative, a disintegrant, a lubricant, a filler, and the like. The liquid formulation includes water, a solution such as a propylene glycol solution, a suspension, an emulsion, and the like, but is not limited thereto, and may be prepared by adding an appropriate colorant, flavoring agent, stabilizer, thickener, and the like. For example, a powder may be prepared by simply mixing a tri-hydroxy derivative of a polysaturated fatty acid, which is an active ingredient of the present invention, with an appropriate pharmaceutically acceptable carrier such as lactose, starch, or microcrystalline cellulose. A granule may be prepared by mixing the tri-hydroxy derivative of the polysaturated fatty acid of the present invention, an appropriate pharmaceutically acceptable carrier, and an appropriate pharmaceutically acceptable binder such as polyvinylpyrrolidone or hydroxypropyl cellulose, and then utilizing a wet granulation method using a solvent such as water, ethanol, or isopropanol, or a dry granulation method using a compressive force. In addition, a tablet may be prepared by mixing the granule with an appropriate pharmaceutically acceptable lubricant such as magnesium stearate, and then tableting the mixture using a tableting machine.

The pharmaceutical composition may be administered in the form of an oral agent, an injection (for example, an intramuscular injection, an intraperitoneal injection, an intravenous injection, an infusion, a subcutaneous injection, or an implant), an inhalation agent, a nasal administration agent, a vaginal agent, a rectal administration agent, a sublingual agent, a transdermal agent, a topical agent, or the like, depending on a disease to be treated and a condition of an individual, but is not limited thereto. Depending on a route of administration, the pharmaceutical composition may be formulated into an appropriate dosage unit formulation containing a pharmaceutically acceptable carrier, additive, and vehicle, which are commonly used and non-toxic.

The pharmaceutical composition may be administered at a daily dosage of about 0.0001 mg/kg to about 10 g/kg, and may be administered at a daily dosage of about 0.001 mg/kg to about 1 g/kg. However, the dosage may vary depending on a degree of purification of the mixture, a patient's condition (age, gender, weight, or the like), and severity of the condition being treated. If necessary, for convenience, the total daily dosage may be administered in divided doses several times throughout a day.

Still another aspect of the present invention provides a method for preventing or treating cancer, the method including treating an individual with the immune cell.

Still another aspect of the present invention provides a use of the immune cell for preventing or treating cancer.

Hereinafter, the present invention will be described in detail with reference to examples.

However, the following examples are intended to illustrate the present invention specifically, and the present invention is not limited by the following examples.

### [Example 1]

### [1-1] Design of single-chain variable fragment of antibody that specifically binds to PD-L1

Five types of anti-PD-L1 antibodies (C4, E7, E8, C8, and E1) capable of specifically binding to programmed death ligand 1 (PD-L1) protein expressed in cancer cells were designed, and a single-chain variable fragment (scFv) of each of the five types of antibodies was designed.

E1 scFv was designed to include a heavy chain variable region (V_{H}) containing a heavy chain CDR1 containing an amino acid sequence of SEQ ID NO: 11, a heavy chain CDR2 containing an amino acid sequence of SEQ ID NO: 12, and a heavy chain CDR3 containing an amino acid sequence of SEQ ID NO: 7, and a light chain variable region (V_{L}) containing a light chain CDR1 containing an amino acid sequence of SEQ ID NO: 13, a light chain CDR2 containing an amino acid sequence of SEQ ID NO: 14, and a light chain CDR3 containing an amino acid sequence of SEQ ID NO: 15.

C4 scFv was designed to include a heavy chain variable region (V_{H}) containing a heavy chain CDR1 containing an amino acid sequence of SEQ ID NO: 16, a heavy chain CDR2 containing an amino acid sequence of SEQ ID NO: 17, and a heavy chain CDR3 containing an amino acid sequence of SEQ ID NO: 3, and a light chain variable region (V_{L}) containing a light chain CDR1 containing an amino acid sequence of SEQ ID NO: 18, a light chain CDR2 containing an amino acid sequence of SEQ ID NO: 19, and a light chain CDR3 containing an amino acid sequence of SEQ ID NO: 20.

E7 scFv was designed to include a heavy chain variable region (V_{H}) containing a heavy chain CDR1 containing an amino acid sequence of SEQ ID NO: 21, a heavy chain CDR2 containing an amino acid sequence of SEQ ID NO: 22, and a heavy chain CDR3 containing an amino acid sequence of SEQ ID NO: 4, and a light chain variable region (V_{L}) containing a light chain CDR1 containing an amino acid sequence of SEQ ID NO: 23, a light chain CDR2 containing an amino acid sequence of SEQ ID NO: 24, and a light chain CDR3 containing an amino acid sequence of SEQ ID NO: 25.

E8 scFv was designed to include a heavy chain variable region (V_{H}) containing a heavy chain CDR1 containing an amino acid sequence of SEQ ID NO: 26, a heavy chain CDR2 containing an amino acid sequence of SEQ ID NO: 27, and a heavy chain CDR3 containing an amino acid sequence of SEQ ID NO: 5, and a light chain variable region (V_{L}) containing a light chain CDR1 containing an amino acid sequence of SEQ ID NO: 28, a light chain CDR2 containing an amino acid sequence of SEQ ID NO: 29, and a light chain CDR3 containing an amino acid sequence of SEQ ID NO: 30.

C8 scFv was designed to include a heavy chain variable region (V_{H}) containing a heavy chain CDR1 containing an amino acid sequence of SEQ ID NO: 31, a heavy chain CDR2 containing an amino acid sequence of SEQ ID NO: 32, and a heavy chain CDR3 containing an amino acid sequence of SEQ ID NO: 6, and a light chain variable region (V_{L}) containing a light chain CDR1 containing an amino acid sequence of SEQ ID NO: 33, a light chain CDR2 containing an amino acid sequence of SEQ ID NO: 34, and a light chain CDR3 containing an amino acid sequence of SEQ ID NO: 35. The CDR sequences of the five types of anti-PD-L1 antibody (C4, E7, E8, C8, and E1) scFvs are as shown in Tables 1 to 5.

**[Table 1]**

| E1 scFv | SEQ ID NO: | Sequence information | Description |
|---|---|---|---|
| | 11 | DYEMS | Heavy chain variable region (V_{H}) CDR1 |
| | 12 | AISGSSGSYTYYADSVKG | Heavy chain variable region (V_{H}) CDR2 |
| | 7 | DRIWYQGSGFDI | Heavy chain variable region (V_{H}) CDR3 |
| | 13 | RASQSISSWLN | Light chain variable region (V_{L}) CDR1 |
| | 14 | AASNLQS | Light chain variable region (V_{L}) CDR2 |
| | 15 | QQSYSFPWT | Light chain variable region (V_{L}) CDR3 |

**[Table 2]**

| C4 scFv | SEQ ID NO: | Sequence information | Description |
|---|---|---|---|
| | 16 | DYAMS | Heavy chain variable region (V_{H}) CDR1 |
| | 17 | AISQSGGTIYYADSVKG | Heavy chain variable region (V_{H}) CDR2 |
| | 3 | GRGRVLWTTFDH | Heavy chain variable region (V_{H}) CDR3 |
| | 18 | RASQDISNWLN | Light chain variable region (V_{L}) CDR1 |
| | 19 | ATSRLQS | Light chain variable region (V_{L}) CDR2 |
| | 20 | QQTYSTPLT | Light chain variable region (V_{L}) CDR3 |

**[Table 3]**

| E7 scFv | SEQ ID NO: | Sequence information | Description |
|---|---|---|---|
| | 21 | SYAMN | Heavy chain variable region (V_{H}) CDR1 |
| | 22 | RISSSGGYTYYADSVKG | Heavy chain variable region (V_{H}) CDR2 |
| | 4 | STVSSLQRGFDL | Heavy chain variable region (V_{H}) CDR3 |
| | 23 | RASQSISNYLN | Light chain variable region (V_{L}) CDR1 |
| | 24 | ATSSLQS | Light chain |
| | | | variable region (V_{L}) CDR2 |
| | 25 | QQSYTFPWT | Light chain variable region (V_{L}) CDR3 |

**[Table 4]**

| E8 scFv | SEQ ID NO: | Sequence information | Description |
|---|---|---|---|
| | 26 | NYGMH | Heavy chain variable region (V_{H}) CDR1 |
| | 27 | AISSSGGSTYYADSVKG | Heavy chain variable region (V_{H}) CDR2 |
| | 5 | RRTLVSAFDV | Heavy chain variable region (V_{H}) CDR3 |
| | 28 | RASQSISNWLN | Light chain variable region (V_{L}) CDR1 |
| | 29 | AASSLQS | Light chain variable region (V_{L}) CDR2 |
| | 30 | QQSYSFPWT | Light chain variable region (V_{L}) CDR3 |

**[Table 5]**

| C8 scFv | SEQ ID NO: | Sequence information | Description |
|---|---|---|---|
| | 31 | DYAMH | Heavy chain variable region (V_{H}) CDR1 |
| | 32 | GISSSGSRKYYADSVKG | Heavy chain variable region (V_{H}) CDR2 |
| | 6 | RGSSRGAFDY | Heavy chain variable region (V_{H}) CDR3 |
| | 33 | RASQDIGNYLN | Light chain variable region (V_{L}) CDR1 |
| | 34 | AASRLQS | Light chain variable region (V_{L}) CDR2 |
| | 35 | QQSYSTPYT | Light chain variable region (V_{L}) CDR3 |

### [1-2] Design of chimeric antigen receptor introduced into natural killer cell

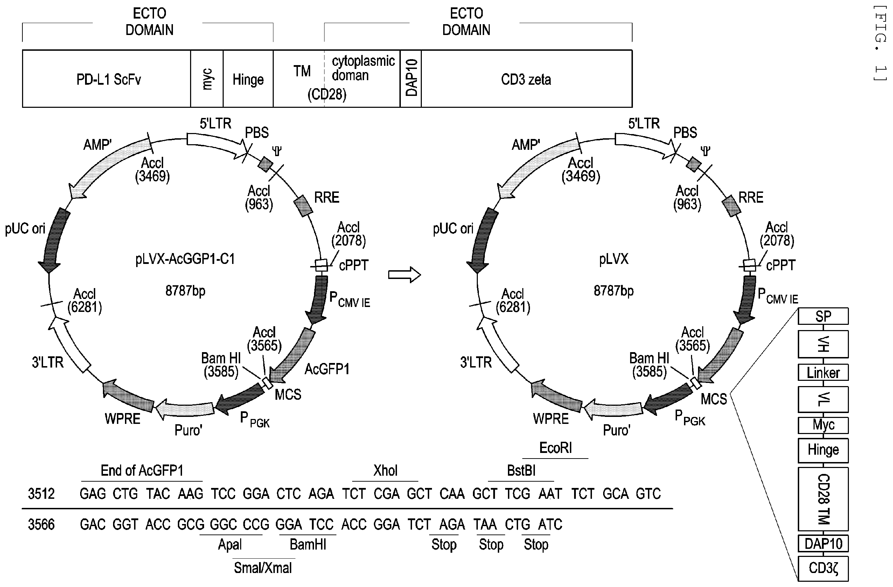

A chimeric antigen receptor (CAR) was designed to contain an extracellular binding domain containing the five types of scFvs designed as described above as antigen binding sites and to be introduced into a natural killer cell. Specifically, the chimeric antigen receptor of the present invention was designed as follows: The extracellular binding domain containing the scFv of the E1 as an antigen binding site was linked to CD28 as a transmembrane domain via Myc and a hinge domain. Additionally, by adding and linking CD3-zeta as an intracellular signaling domain and DAP10 as a co-stimulatory molecule to the transmembrane domain, the chimeric antigen receptors of the present invention were designed to contain the intracellular signaling domain of a a chimeric antigen receptor classified as a third-generation CAR (referred to as "PD-L1_E1 CAR", "PD-L1_C4 CAR", "PD-L1_E7 CAR", "PD-L1_E8 CAR", and "PD-L1_C8 CAR", and collectively referred to as "PD-L1 CAR") (see FIG. 1A). Then, a base sequence of a gene construct encoding the same was used as a backbone of a lentiviral expression vector pLVX-AcGFP1-C1, an AcGFP region was deleted, and then, a gene construct encoding the chimeric antigen receptor of the present invention was inserted (see FIG. 1B).

### [Example 2]

### Production of immune cell expressing chimeric antigen receptor for PD-L1 on surface thereof

A natural killer cell capable of expressing the chimeric antigen receptors of the present invention designed by Example 1 on a surface thereof was produced.

The lentiviral vector prepared in Example [1-2] was transfected into HEK293T cells together with a viral packaging vector (pMDLG/RRE, pRSV/REV, or VSVG). A lentivirus expressing PD-L1 CAR was obtained therefrom, concentrated using an ultrahigh-speed centrifuge, and then, infected into natural killer cells using a spinoculation method (360 g, 90 min, RT) so that a multiplicity of infection (MOI) was 30. The infected natural killer cells as described above were cultured under conditions of 37°C and 5% CO₂ for 5 hours, the medium was replaced with a fresh medium, and after 3 days, the culture was continued by treating the cells with puromycin at a concentration of 3 ug/ml to select properly infected natural killer cells. As a control group, uninfected natural killer cells were also treated with puromycin, and culture was continued using a medium treated with puromycin until all natural killer cells in the control group were killed by puromycin. Experiments were performed by selecting natural killer cells infected at the point when all natural killer cells in the control group were killed.

The natural killer cells expressing "PD-L1_E1 CAR", "PD-L1_C4 CAR", "PD-L1_E7 CAR", "PD-L1_E8 CAR", and "PD-L1_C8 CAR" produced and selected as described above (referred to as "PD-L1_E1 CAR NK cells", "PD-L1_C4 CAR NK cells", "PD-L1_E7 CAR NK cells", "PD-L1_E8 CAR NK cells", and "PD-L1_C8 CAR NK cells", respectively, and collectively referred to as "PD-L1 CAR-NK cells") were treated with an anti-myc antibody (CST; 9B11) that specifically binds to myc of PD-L1 CAR (4°C, in the dark for 30 minutes), and the expression of Myc was confirmed by flow cytometry. At this time, the original natural killer cells not expressing PD-L1 CAR were used as a control group.

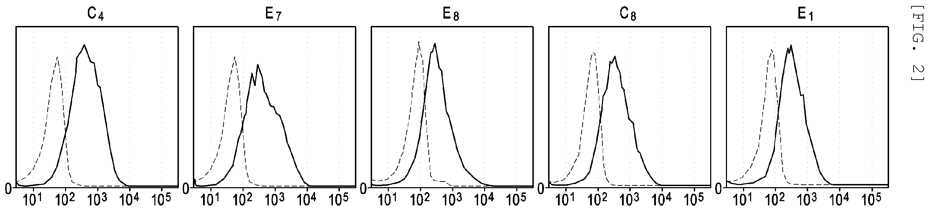

As a result, as illustrated in FIG. 2, it was confirmed that PD-L1 CAR was properly expressed in all the five types of PD-L1 CAR NK cells compared to the control group (see FIG. 2).

### [Example 3]

### Confirmation of cytolytic activity of PD-L1 CAR NK cells against cancer cells expressing PD-L1

As produced in Example 2, a natural killer cell expressing the chimeric antigen receptor of the present invention on a surface thereof contains scFv that specifically binds to PD-L1 as an antigen binding site. Thus, cytolytic activity (cell lysis) against cancer cells expressing PD-L1 was confirmed.

### [3-1] Selection of cancer cells expressing PD-L1

First, MDA-MB-231 cells, which are a breast cancer cell line, were selected as cancer cells expressing PD-L1, and AU565 cells, which are another breast cancer cell line, were selected as cancer cells that do not express PD-L1, the selected cells were treated with an anti-PD-L1 antibody (BD Biosciences) in an amount of 1 ul/100 ul, and a reaction was allowed to proceed (4°C, in the dark for 30 minutes), and expression levels of PD-L1 in the two cell lines were confirmed using a flow cytometer.

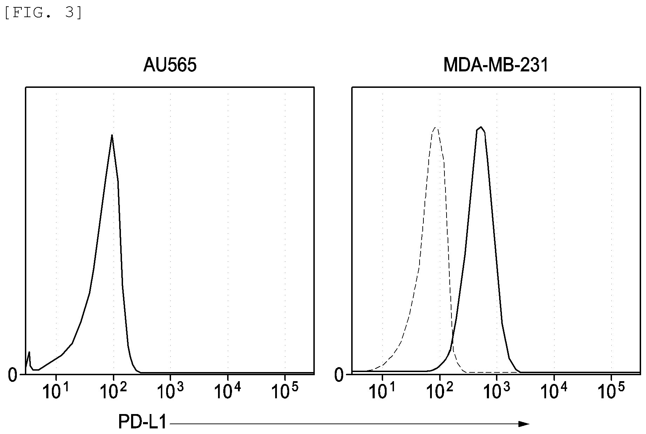

As a result, as illustrated in FIG. 3, it was confirmed that MDA-MB-231 cells expressed PD-L1, and AU565 cells did not express PD-L1 (see FIG. 3).

### [3-2] Confirmation of activity of PD-L1 CAR NK cells against cancer cells expressing PD-L1

For MDA-MB-231 cells and AU565 cells whose PD-L1 expression was confirmed as described above, the cytotoxicity of each of the five types of PD-L1 CAR NK cells produced in Example 2 was confirmed by a calcein AM assay. Specifically, MDA-MB-231 cells and AU565 cells were treated with calcein at a concentration of 5 ug/ml and allowed to react (37°C, 5% CO₂, in the dark for 1 hour), and then, each cancer cell stained with calcein was treated with the original natural killer cells (PURO-92) as a control group and the five types of PD-L1 CAR NK cells. PD-L1_C4 CAR NK, PD-L1_E7 CAR NK, and PD-L1_E8 CAR NK cells were treated at E:T ratios (Effector to Target Ratio, in this case, natural killer cells:cancer cells) of 10:1 and 2:1, respectively, PD-L1_C8 CAR NK and PD-L1_E1 CAR NK cells were treated at E:T ratios of 5:1 and 1:1 and allowed to react (4 hours under 37°C and 5% CO₂ conditions), and 100 ul of a supernatant was taken to confirm the amount of calcein present in the supernatant.

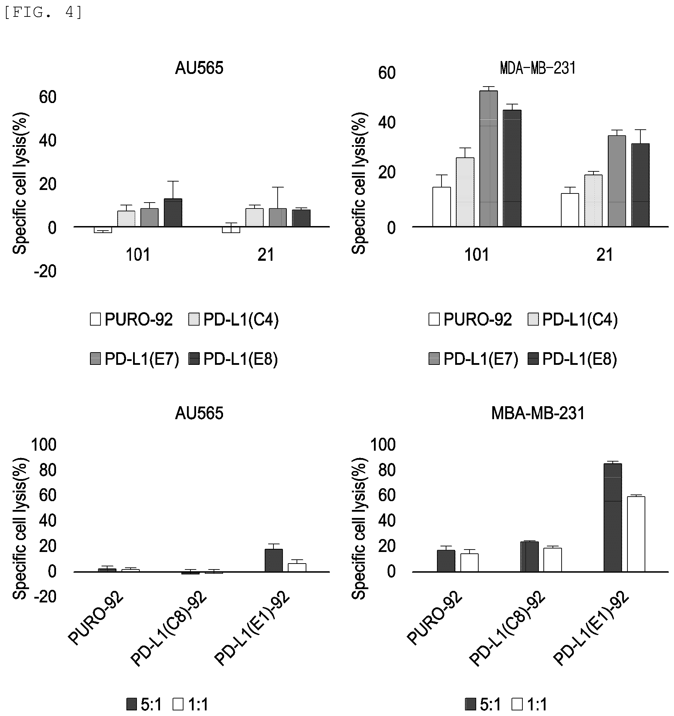

As a result, as illustrated in FIG. 4, all the five types of PD-L1 CAR NK cells exhibited much higher cytotoxicity against MDA-MB-231 cells expressing PD-L1 than the control natural killer cells.

In the case where PD-L1_C4 CAR NK, PD-L1_E7 CAR NK, and PD-L1_E8 CAR NK cells were treated at a ratio of 10:1 or 2:1, cell lysis was observed in more cells when all three types of cells were treated at a ratio of 10:1 compared to the case where the cells were treated at a ratio of 2:1. Meanwhile, in the case of PD-L1_C8 CAR NK cells, there was no significant difference in cell lysis rate when treated at a ratio of 5:1 or 1:1, and in the case of PD-L1_E1 CAR NK cells, a cell lysis rate of 80% or more was observed when treated at a ratio of 5:1, showing the best cell lysis rate among the five types of PD-L1 CAR NK cells (see FIG. 4).

Furthermore, cytokine and granule secretion were confirmed for PD-L1_E1 CAR NK cells, which showed the best cytolysis rate among the five types of PD-L1 CAR NK cells.

Specifically, MDA-MB-231 cells and AU565 cells were treated with natural killer cells into which cot-CAR was introduced as a control group and PD-L1_E1 CAR NK cells as an experimental group at 1:1 and allowed to react (16 hours under 37°C and 5% CO2 conditions), and then, a supernatant was collected to confirm interferon-γ (IFN-γ) present in the supernatant through ELISA. At this time, the amount of cytokines secreted solely from natural killer cells into which cot-CAR was introduced (hereinafter, referred to as "cot-CAR NK cells") and PD-L1_E1 CAR NK cells was used as a control group.

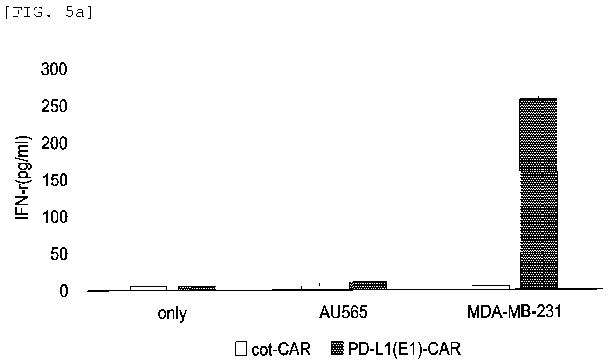

As a result, as illustrated in FIG. 5A, cot-CAR NK and PD-L1_E1 CAR NK cells alone secreted little IFN-γ, and cot-CAR NK and PD-L1_E1 CAR NK cells treated with AU565 cells that do not express PD-L1 also secreted little IFN-γ. However, it was confirmed that the amount of IFN-γ secreted was significantly increased only in PD-L1 CAR_E1 NK cells treated with MDA-MB-231 cells expressing PD-L1 (see FIG. 5A).

In addition, MDA-MB-231 cells or AU565 cells were mixed with cot-CAR NK cells or PD-L1_E1 CAR NK cells as a control group at 1:1 in RPMI (10% FBS), allowed to react (4 hours under 37°C and 5% CO₂ conditions), treated with an anti-CD56 antibody to select natural killer cells, and then, stained, and a degree of CD107a secretion in cot-CAR NK cells and PD-L1_E1 CAR NK cells was analyzed through flow cytometry.

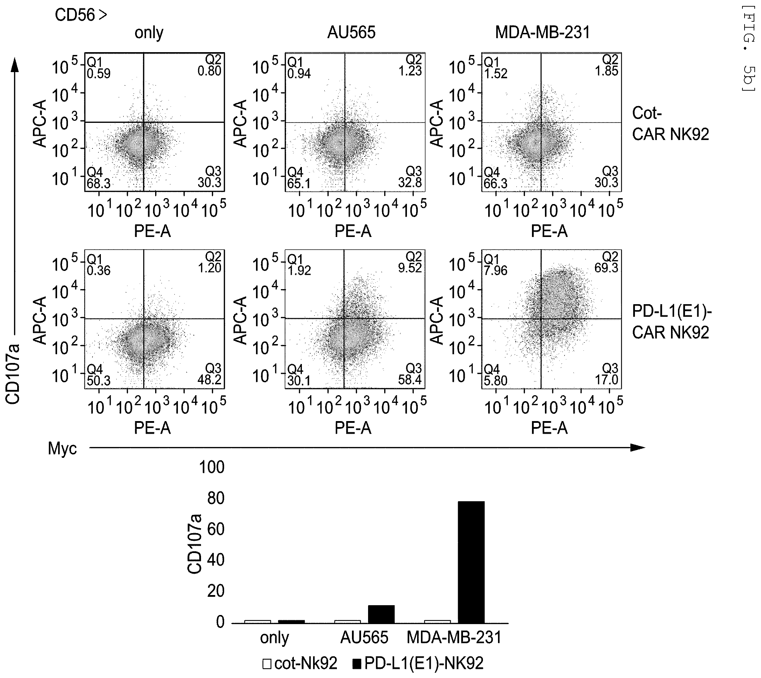

As a result, as illustrated in FIG. 5B, similar to the case of IFN-γ, it was confirmed that secretion of CD107a was significantly increased only in MDA-MB-231 cells expressing PD-L1 (see FIG. 5B).

### [Example 4]

### Confirmation of immune synapse formation patterns of PD-L1 CAR NK cells against cancer cells expressing PD-L1

For PD-L1_E1 CAR NK cells, a formation rate of immune synapses, which are contact points where immune cells come into contact with cancer cells and secrete cytokines and granules important for cancer cell death, and a rate of cancer cell death relative to immune cell contact were confirmed.

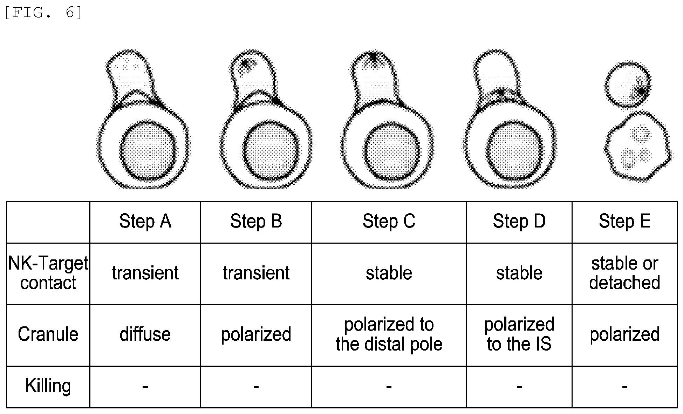

First, a process from the formation of the immune synapses (stage A) to the death of cancer cells (stage E) was divided into a total of five stages including the stages A to E (see FIG. 6).

MDA-MB-231 cell lines expressing PD-L1 were treated with native natural killer cells (PURO-92) as a control group and PD-L1_E1 CAR NK cells, respectively. Specifically, first, PURO-92 cells and PD-L1_E1 CAR NK cells were labeled by a treatment with 1 ug/ml of LysoSensor Green DND-153 (Invitrogen), which is a pH sensing reagent, respectively, and MDA-MB-231 cells were labeled by a reaction with 10 ug/ml of CellTrace Far Red DDAO-SE (ThermoFisher) at 37°C for 15 minutes. Thereafter, the labeled 2 to 5 x 10⁵ PD-L1_E1 CAR NK cells were suspended in a medium containing 10 ug/ml of propidium iodide (BD Biosciences), and the cells were placed on coverslips of Chamlide Magnetic Chamber (Live Cell Instruments) coated with anti-CD44. The PD-L1_E1 NK cells placed on the chamber coverslips were mounted on a live cell microscope and cultured at 37°C for 30 minutes, and then, the labeled MDA-MB-231 cells were added. Time lapse imaging was performed at intervals of 3 minutes.

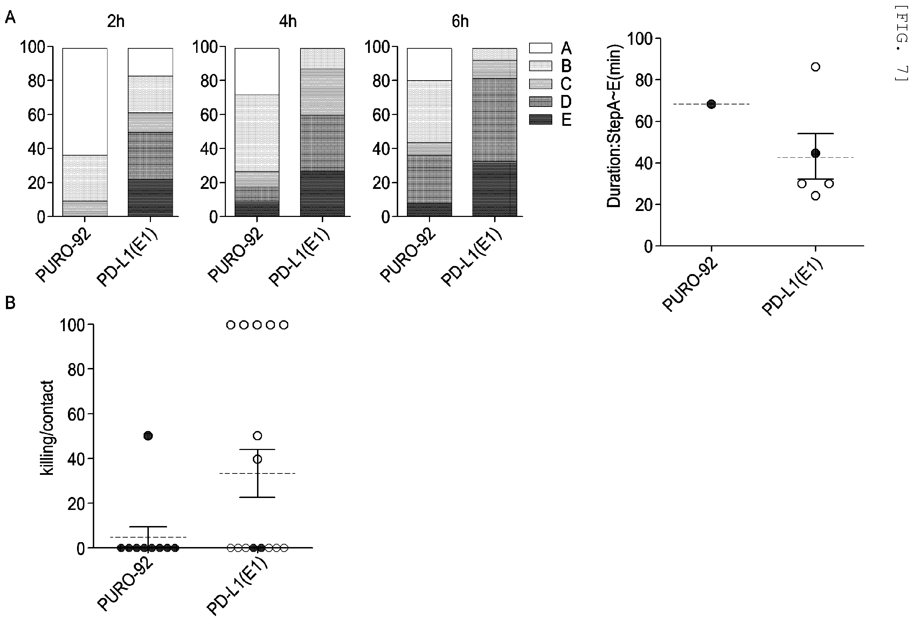

As a result, as illustrated in FIG. 7A, in the case of PD-L1_E1 CAR NK cells, at 2 hours after treatment, about 50% of the immune synapses were in the stage D or the stage E, which is a late stage. However, in the case of PURO-92, no cells were observed in the stage D or the stage E at the same time point. Furthermore, at 6 hours after treatment, when PD-L1_E1 CAR NK cells were treated, about 80% of the immune synapses were in the stage D or the stage E, but, when PURO-92 was treated, only 40% of the cells were in the stage D or the stage E, and among them, only about 10% of the immune synapses were in the stage E, which is the stage of cell death and the final stage of the immune synapse. When comparing the time from the formation of the immune synapses to the cell death, it was confirmed that it took about 170 minutes when PURO-92 was treated, whereas it took about 100 minutes when PD-L1_E1 CAR NK was treated, which showed that a much shorter time was required for the death of cancer cells expressing PD-L1 (see FIG. 7A).

In addition, as illustrated in FIG. 7B, when comparing the rate of cell death relative to contact with MDA-MB-231 cells expressing PD-L1, the rate of cell death relative to contact did not even reach about 0.1 when PURO-92 was treated, whereas the rate reached about 0.4 when PD-L1_E1 CAR NK was treated, which showed a targeting specificity that was about 4 times or more higher (see FIG. 7B).

### [Example 5]

### Confirmation of in-vivo apoptotic activity of PD-L1 CAR NK cells against cancer cells expressing PD-L1

### [5-1] Selection of lung cancer cell lines expressing PD-L1 and confirmation of PD-L1 CAR NK cell activity against them

It was intended to determine whether PD-L1 CAR NK cells had cell apoptotic activity also in vivo against cancer cells expressing PD-L1.

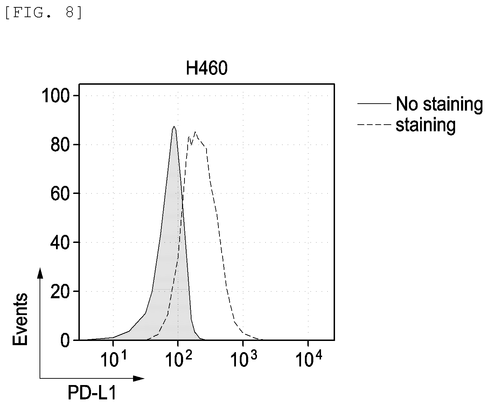

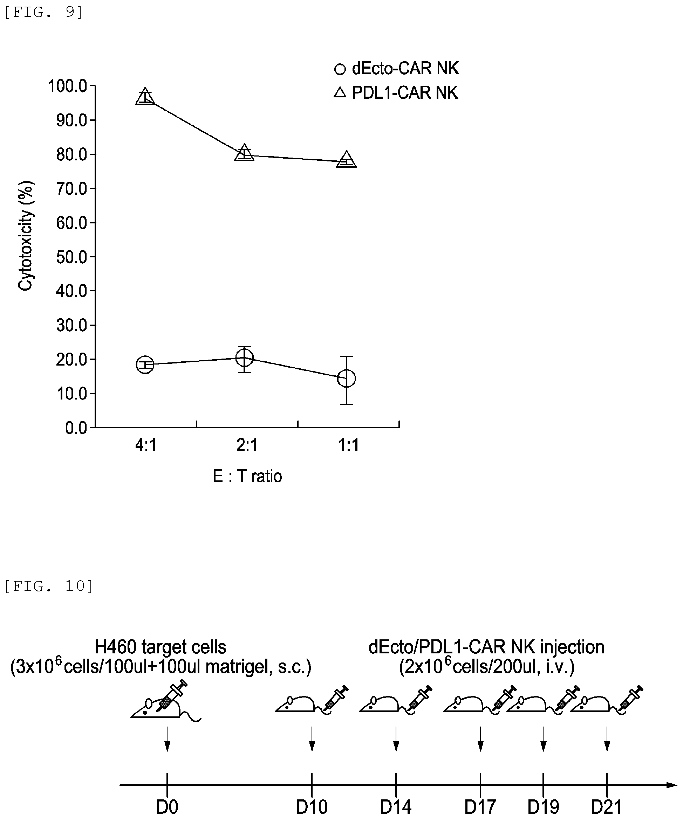

H460, which is a lung cancer cell line, was selected as a cancer cell expressing PD-L1, and it was confirmed whether PD-L1 was expressed on the surface of the H460 cell using the method described in Example [3-1] (see FIG. 8). Thereafter, H460 cells were treated with PD-L1_E1 CAR NK cells as an experimental group and dEcto CAR NK cells as a control group at E:T ratios of 4:1, 2:1, and 1:1 in the same manner as described in Example [3-2]. As a result, the rate of cell death was significantly higher when the cells of the experimental group were treated than when the cells of the control group were treated. In addition, the rate of cell death was concentration-dependent, and in particular, when treated at an E:T ratio of 4:1, the rate of cell death reached almost 100% (see FIG. 9). Accordingly, the activity of PD-L1 CAR NK cells against the lung cancer cell line H460 was also confirmed.

### [5-2] Confirmation of in-vivo anticancer activity of PD-L1 CAR NK cells against lung cancer cell line expressing PD-L1

In order to confirm the in-vivo anticancer activity, the changes in tumor size and weight of the H460 cell line when PD-L1_E1 CAR NK cells were injected into mice were confirmed.

Specifically, on day 0, H460 lung cancer cell line mixed with 100 ul of Matrigel at a ratio of 1:1 was subcutaneously injected into approximately 6-week-old female nude mice (BALB/c) (Saeron Bio Inc.) at a concentration of 3 × 10⁶ cells/100 ul. Thereafter, on day 10, dEcto CAR NK cells as a control group and PD-L1 CAR NK cells as an experimental group were each intravenously injected at a concentration of 2 × 10⁶ cells/200 ul on day 10, day 14, day 17, day 19, and day 21 (see FIG. 10).

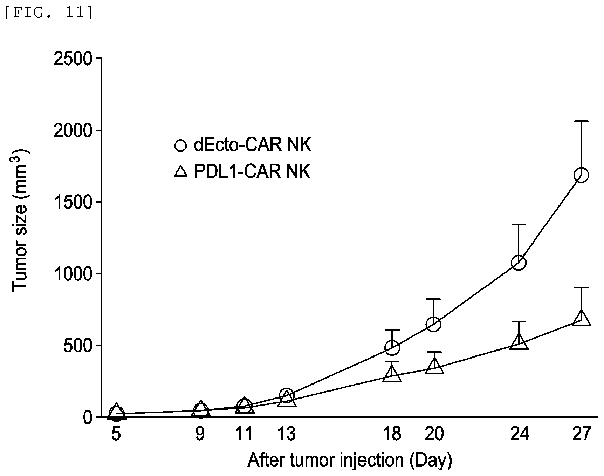

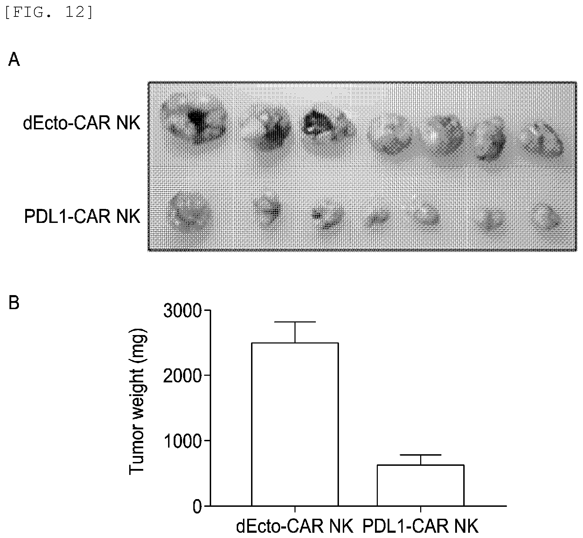

Cancer cells and NK cells as a control group and an experimental group were injected using the above method, and the tumor sizes were compared at intervals of 2 to 5 days from day 5 to day 27. Until 13 days had elapsed, the tumor sizes were similar in the case where the NK cells as the control group or the experimental group were injected, but from day 18, a significant difference in tumor size began to be observed in the case where the NK cells as the experimental group were injected, and after day 27, the tumor size was about 500 mm³ in the case where the NK cells as the experimental group were injected, whereas there was a difference in tumor volume exceeding 1,500 mm³, which was about 3 times larger, in the case where the NK cells as the control group were injected (see FIG. 11). In addition, even in a case where the weight of the tumor (see FIG. 12A) was measured after day 32, the weight was only about 700 mg when the NK cells as the experimental group were injected, but the weight reached 2,500 mg when the NK cells as the control group were injected (see FIG. 12B).

Although the present invention has been described in detail above by way of only the described embodiments, it will be apparent to those skilled in the art that various changes and modifications are possible within the technical scope of the present invention, and it is also natural that such changes and modifications fall within the scope of the appended claims.

## Claims

1. An antibody or an antigen-binding fragment thereof that specifically binds to programmed death ligand 1 (PD-L1), the antibody or the antigen-binding fragment thereof comprising:
a heavy chain variable region comprising a heavy chain CDR1 comprising an amino acid sequence of X1-Y-X2-M-X3 (SEQ ID NO: 1), a heavy chain CDR2 comprising an amino acid sequence of X₄-I-S-X₅-S-G-X₆-X₇-X_{B}-Y-Y-A-D-S-V-K-G (SEQ ID NO: 2), and a heavy chain CDR3 comprising any one sequence selected from the group consisting of SEQ ID NO: 3, SEQ ID NO: 4, SEQ ID NO: 5, SEQ ID NO: 6, and SEQ ID NO: 7; and
a light chain variable region comprising a light chain CDR1 comprising an amino acid sequence of R-A-S-Q-X₉-I-X₁₀-X₁₁-X₁₂-L-N (SEQ ID NO: 8), a light chain CDR2 comprising an amino acid sequence of A-X₁₃-S-X₁₄-L-Q-S (SEQ ID NO: 9), and a light chain CDR3 comprising an amino acid sequence of Q-Q-X₁₅-Y-X₁₆-X₁₇-P-X₁₈-T (SEQ ID NO: 10),
(wherein each of X₁ to X₁₈ in the amino acid sequences of SEQ ID NO: 1, SEQ ID NO: 2, and SEQ ID NOs: 8 to 10is independently any amino acid).

2. The antibody or the antigen-binding fragment thereof of claim 1,
wherein X₁ is any one selected from the group consisting of aspartic acid, glutamic acid, serine, threonine, asparagine, and glutamine;
X₂ is any one selected from the group consisting of alanine, leucine, isoleucine, glycine, aspartic acid, and glutamic acid;
X₃ is any one selected from the group consisting of serine, threonine, asparagine, glutamine, histidine, arginine, and lysine;
X₄ is any one selected from the group consisting of alanine, leucine, isoleucine, arginine, histidine, lysine, glycine, alanine, leucine, and isoleucine;
X₅ is any one selected from the group consisting of serine, threonine, glutamine, asparagine, glycine, alanine, leucine, and isoleucine;
X₆ is any one selected from the group consisting of glycine, alanine, leucine, isoleucine, serine, and threonine;
X₇ is any one selected from the group consisting of threonine, serine, tyrosine, phenylalanine, tryptophan, serine, threonine, arginine, histidine, and lysine;
X₈ is any one selected from the group consisting of alanine, leucine, isoleucine, serine, threonine, arginine, histidine, and lysine;
X₉ is any one selected from the group consisting of aspartic acid, glutamic acid, serine, and threonine;
X₁₀ is any one selected from the group consisting of serine, threonine, glycine, alanine, leucine, and isoleucine;
X₁₁ is any one selected from the group consisting of asparagine, glutamine, serine, and threonine;
X₁₂ is any one selected from the group consisting of tryptophan, tyrosine, and phenylalanine;
X₁₃ is any one selected from the group consisting of serine, threonine, glycine, alanine, leucine, and isoleucine;
X₁₄ is any one selected from the group consisting of arginine, histidine, lysine, serine, threonine, asparagine, and glutamine;
X₁₅ and X₁₆ are each independently any one selected from the group consisting of serine and threonine;
X₁₇ is any one selected from the group consisting of serine, threonine, phenylalanine, tryptophan, and tyrosine; or
X₁₈ is any one selected from the group consisting of leucine, isoleucine, phenylalanine, tryptophan, and tyrosine.

3. The antibody or the antigen-binding fragment thereof of claim 1,
wherein X₁ is any one selected from the group consisting of aspartic acid, serine, and asparagine;
X₂ is any one selected from the group consisting of alanine, glycine, and glutamic acid;
X₃ is any one selected from the group consisting of serine, asparagine, and histidine;
X₄ is any one selected from the group consisting of alanine, arginine, and glycine;
X₅ is any one selected from the group consisting of serine, glutamine, and glycine;
X₆ is any one selected from the group consisting of glycine and serine;
X₇ is any one selected from the group consisting of threonine, tyrosine, serine, and arginine;
X₈ is any one selected from the group consisting of isoleucine, threonine, and lysine;
X₉ is any one selected from the group consisting of aspartic acid and serine;
X₁₀ is any one selected from the group consisting of serine and glycine;
X₁₁ is any one selected from the group consisting of asparagine and serine;
X₁₂ is any one selected from the group consisting of tryptophan and tyrosine;
X₁₃ is any one selected from the group consisting of threonine and alanine;
X₁₄ is any one selected from the group consisting of arginine, serine, and asparagine;
X₁₅ and X₁₆ are each independently any one selected from the group consisting of serine and threonine;
X₁₇ is any one selected from the group consisting of threonine and phenylalanine; or
X₁₈ is any one selected from the group consisting of leucine, tryptophan, and tyrosine.

4. The antibody or the antigen-binding fragment thereof of claim 1,
wherein the heavy chain CDR1 is any one selected from the group consisting of amino acid sequences of SEQ ID NO: 11, SEQ ID NO: 16, SEQ ID NO: 21, SEQ ID NO: 26, and SEQ ID NO: 31;
the heavy chain CDR2 is any one selected from the group consisting of amino acid sequences of SEQ ID NO: 12, SEQ ID NO: 17, SEQ ID NO: 22, SEQ ID NO: 27, and SEQ ID NO: 32;
the heavy chain CDR3 is any one selected from the group consisting of amino acid sequences of SEQ ID NO: 3, SEQ ID NO: 4, SEQ ID NO: 5, SEQ ID NO: 6, and SEQ ID NO: 7;
the light chain CDR1 is any one selected from the group consisting of amino acid sequences of SEQ ID NO: 13, SEQ ID NO: 18, SEQ ID NO: 23, SEQ ID NO: 28, and SEQ ID NO: 33;
the light chain CDR2 is any one selected from the group consisting of amino acid sequences of SEQ ID NO: 14, SEQ ID NO: 19, SEQ ID NO: 24, SEQ ID NO: 29, and SEQ ID NO: 34; or
the light chain CDR3 is any one selected from the group consisting of amino acid sequences of SEQ ID NO: 15, SEQ ID NO: 20, SEQ ID NO: 25, SEQ ID NO: 30, and SEQ ID NO: 35.

5. The antibody or the antigen-binding fragment thereof of claim 1, wherein the heavy chain CDR1 comprises an amino acid sequence of SEQ ID NO: 11;
the heavy chain CDR2 comprises an amino acid sequence of SEQ ID NO: 12;
the heavy chain CDR3 comprises an amino acid sequence of SEQ ID NO: 7;
the light chain CDR1 comprises an amino acid sequence of SEQ ID NO: 13;
the light chain CDR2 comprises an amino acid sequence of SEQ ID NO: 14; or
the light chain CDR3 comprises an amino acid sequence of SEQ ID NO: 15.

6. A chimeric antigen receptor (CAR) comprising:
an extracellular binding domain comprising an antigen binding site that specifically binds to PD-L1;
a transmembrane domain; and
an endodomain,
wherein the antigen binding site that specifically binds to PD-L1 is a single-chain variable fragment (ScFv) of the antibody or the antigen-binding fragment thereof of any one of claims 1 to 5.

7. The chimeric antigen receptor of claim 6, wherein the extracellular binding domain further comprises at least one selected from the group consisting of a hinge domain and a myc domain.

8. The chimeric antigen receptor of claim 6, wherein the endodomain is at least one selected from the group consisting of a DAP10 domain and a Cd3zeta domain.

9. A polynucleotide comprising a base sequence encoding the chimeric antigen receptor of claim 6.

10. An expression vector comprising the polynucleotide of claim 9.

11. An immune cell expressing the chimeric antigen receptor of claim 6 on a surface thereof.

12. The immune cell of claim 11, wherein the immune cell is any one selected from the group consisting of a natural killer cell (NK cell), a T cell, a natural killer T cell (NKT cell), a cytokine induced killer cell (CIK), a macrophage, and a dendritic cell.

13. A pharmaceutical composition for treating or preventing cancer, the pharmaceutical composition comprising the immune cell of claim 11.

14. The pharmaceutical composition of claim 13, wherein the cancer expresses PD-L1 on a surface of a cancer cell.

15. The pharmaceutical composition of claim 14, wherein the cancer is at least one selected from the group consisting of lung cancer, stomach cancer, ovarian cancer, cervical cancer, breast cancer, pancreatic cancer, colon cancer, colorectal cancer, esophageal cancer, skin cancer, thyroid cancer, kidney cancer, liver cancer, head and neck cancer, bladder cancer, prostate cancer, blood cancer, multiple myeloma, acute myeloid leukemia, malignant lymphoma, thymus cancer, osteosarcoma, fibrous tumor, and brain cancer.
